(19) Europäisches Patentamt
European Patent Office
Office européen des brevets

(11) **EP 3 931 523 B1**

(12) **EUROPÄISCHE PATENTSCHRIFT**

(45) Veröffentlichungstag und Bekanntmachung des Hinweises auf die Patenterteilung:
**13.08.2025 Patentblatt 2025/33**

(21) Anmeldenummer: **20711801.9**

(22) Anmeldetag: **28.02.2020**

(51) Internationale Patentklassifikation (IPC):
**G01B 9/02** (2022.01)    **H01S 5/042** (2006.01)
**H01S 5/06** (2006.01)    **H01S 5/062** (2006.01)

(52) Gemeinsame Patentklassifikation (CPC):
**G01B 9/02004; G01B 9/02091; H01S 5/0622;**
**H01S 5/0654;** H01S 5/06804; H01S 5/0687;
H01S 5/183; H01S 5/34326

(86) Internationale Anmeldenummer:
**PCT/EP2020/055291**

(87) Internationale Veröffentlichungsnummer:
**WO 2020/174088 (03.09.2020 Gazette 2020/36)**

(54) **VERFAHREN ZUR STEUERUNG EINES HALBLEITERLASERDIODEN-BASIERTEN SS-INTERFEROMETER-SYSTEMS**

METHOD FOR CONTROLLING A SEMICONDUCTOR-LASER-DIODE-BASED SS-INTERFEROMETER SYSTEM

PROCÉDÉ DE COMMANDE D'UN SYSTÈME D'INTERFÉROMÈTRE SS À BASE DE DIODES LASER À SEMICONDUCTEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.02.2019 DE 102019202739**

(43) Veröffentlichungstag der Anmeldung:
**05.01.2022 Patentblatt 2022/01**

(73) Patentinhaber: **Carl Zeiss Meditec AG**
**07745 Jena (DE)**

(72) Erfinder:
- **LEITGEB, Rainer**
  **1030 Wien (AT)**
- **DICK, Manfred**
  **07926 Gefell (DE)**
- **BERGNER, Roland**
  **07745 Jena (DE)**
- **EBERSBACH, Ralf**
  **04626 Schmölln (DE)**
- **PABST, Thomas**
  **07646 Stadtroda (DE)**

(74) Vertreter: **Kintzel, Klaus-Peter**
**Carl Zeiss AG**
**Patentabteilung**
**Carl-Zeiss-Promenade 10**
**07745 Jena (DE)**

(56) Entgegenhaltungen:
- **IRENEUSZ GRULKOWSKI ET AL: "Retinal, anterior segment and full eye imaging using ultrahigh speed swept source OCT with vertical-cavity surface emitting lasers", BIOMEDICAL OPTICS EXPRESS, vol. 3, no. 11, 1 November 2012 (2012-11-01), pages 2733 - 2751, XP055133476, ISSN: 2156-7085, DOI: 10.1364/ BOE.3.002733**
- **SUCBEI MOON ET AL: "VCSEL-based swept source for low-cost optical coherence tomography", BIOMEDICAL OPTICS EXPRESS, vol. 8, no. 2, 1 February 2017 (2017-02-01), United States, pages 1110, XP055702048, ISSN: 2156-7085, DOI: 10.1364/BOE.8.001110**

- "Communications in computer and information science", vol. 894, 1 January 2018, SPRINGER, DE, ISSN: 1865-0929, article YING XU ET AL: "Improving the Resolution of Retinal OCT with Deep Learning : 22nd Conference, MIUA 2018, Southampton, UK, July 9-11, 2018, Proceedings", pages: 325 - 332, XP055703789, DOI: 10.1007/978-3-319-95921-4_30
- KOICHI IIYAMA ET AL: "High-Resolution FMCW Reflectometry Using a Single-Mode Vertical-Cavity Surface-Emitting Laser", IEEE PHOTONICS TECHNOLOGY LETTERS, IEEE SERVICE CENTER, PISCATAWAY, NJ, US, vol. 23, no. 11, 1 June 2011 (2011-06-01), pages 703 - 705, XP011480009, ISSN: 1041-1135, DOI: 10.1109/LPT.2011.2131124
- HEFFERMAN GERALD ET AL: "Extended-bandwidth frequency sweeps of a distributed feedback laser using combined injection current and temperature modulation", REVIEW OF SCIENTIFIC INSTRUMENTS, AIP, MELVILLE, NY, US, vol. 88, no. 7, 27 July 2017 (2017-07-27), XP012220422, ISSN: 0034-6748, [retrieved on 20170727], DOI: 10.1063/1.4991817

**Beschreibung**

Die vorliegende Erfindung betrifft ein Verfahren zur Steuerung eines Halbleiterlaserdioden-basierten SS-Interferometer-Systems (SS = swept source), welches eine breite Anwendung ermöglicht. Weiterhin soll das System für die Verwendung in der Ophthalmologie, insbesondere zur Bildgebung und zur Bestimmung biometrischer Messwerte des Auges, geeignet sein.

Ein Interferometer ist ein technisches optisches Gerät, das die Interferenzen für Präzisionsmessungen nutzt. Gemessen werden alle Effekte, die die effektive Weglänge der Wellen und damit Eigenschaften der überlagerten Welle ändern.

Beispiele dafür sind Längenänderungen eines der beiden überlagerten Lichtwege zur Längenmessung, Änderungen des Brechungsindex zur Messung von Materialeigenschaften oder minimale Änderungen des Abstandes zwischen den Testmassen in Gravitationswellendetektoren.

Dem entsprechend sind nach dem Stand der Technik zahlreiche Lösungen bekannt.

Eine spezielle interferometrische Anwendung stellt die optische Kohärenztomographie (OCT = optical coherence tomography), als ein bildgebendes Verfahren dar. Mittels OCT-Systemen ist es möglich 2- und 3-dimensionale Aufnahmen streuender Materialien in Mikrometerauflösung zu erhalten. Haupteinsatzgebiet der OCT ist die Medizin, insbesondere die Ophthalmologie.

Bei den OCT-Verfahren wird kohärentes Licht mit Hilfe eines Interferometers zur Entfernungsmessung und Bildgebung an reflexiven und streuenden Proben eingesetzt. Am menschlichen Auge liefern die OCT-Verfahren beim Scan in die Tiefe, aufgrund der an optischen Grenzflächen auftretenden Änderungen des Brechungsindexes und aufgrund von Volumenstreuung, messbare Signale. Bei der optischen Kohärenztomographie handelt es sich um ein sehr empfindliches und schnelles Verfahren.

Um dabei die Messwerterfassung noch effektiver zu gestalten, fanden in den letzten Jahren OCT-Systeme Verwendung, die auf der sogenannten "Swept Source"-Technik (SS-OCT) basieren. Hierbei wird die Frequenz der Lichtquelle durchgestimmt und dadurch die Tiefensignale erzeugt. Diese Technik erlaubt die Durchführung von Ganzaugenscans am menschlichen Auge. Dafür ist allerdings eine entsprechende Auswahl und Steuerung der Beleuchtungsquelle erforderlich.

Aktuelle SS-OCT-Systeme benutzen komplexe mikroelektromechanische Laserdioden Systeme (MEMS), um spektrale Laserlinien mit einer hohen Kohärenzlänge (im Bereich von cm bis m) mit einer hohen Wiederholrate (im Bereich von kHz bis MHz) über einen breiten Wellenlängenbereich von bis zu 150nm durchzustimmen. Dies ist erforderlich, um über eine hohe Messtiefe sehr schnell hochaufgelöste Abbildungen insbesondere in transparentem organischem Gewebe, wie dem menschlichen Auge, mit einer hohen axialen Auflösung zu erhalten.

Die Kohärenzlänge ist in der Optik der maximale Weglängen- oder Laufzeitunterschied, den zwei Lichtstrahlen aus derselben Quelle haben dürfen, damit bei ihrer Überlagerung noch ein (räumlich und zeitlich) stabiles Interferenzmuster entsteht.

Die Detektion bei OCT-Systemen kann natürlich nicht rauschfrei erfolgen. Somit kann eine Reflexion in der Probe nur detektiert werden, wenn sie ein Signal liefert, welches größer als der Rauschuntergrund im OCT-System ist.

Diese kleinste noch detektierbare Reflexion ist eine sehr wichtige Kenngröße von OCT-Systemen und wird als Sensitivität bezeichnet und üblicherweise in dB angegeben.

Bei den Betrachtungen der Sensitivität ist weiterhin der sogenannte "Sensitivity Roll-Off"-Kriterium zu berücksichtigen, unter dem man die Abnahme der Amplitude des Interferenz-Signals mit zunehmendem Längen-Unterschied zwischen Referenz- und Probenarm versteht.

Für die hier beschriebenen OCT-Systeme wird eine Sensitivität (unter Berücksichtigung des "Sensitivity Roll-Off"-Kriteriums) von -6 dB definiert.

Nach dem bekannten Stand der Technik gibt es aber auch schon Bestrebungen und Versuche zur Verwendung anderer Laserdioden, beispielsweise VCSEL (Vertical Cavity Surface Emitting Laser). Einen Überblick kann man dem Artikel "Ultra-Widely Tunable VCSELs" von Garrett D. Cole in [1] entnehmen.

Beispielhaft wird hierzu auf eine single-mode (auch mono-mode) VCSEL-Laserdiode von Philips verwiesen, deren technische Daten in [3] beschrieben werden. Diese, über 2nm durchstimmbare VCSEL-Laserdiode kann thermoelektrisch in einem Temperaturbereich von 10 - 40°C bei dann leicht unterschiedlicher Mittenwellenlänge betrieben werden.

Von Sucbei Moon und Eun Seo Choi werden in [4] low-cost OCT-Systeme beschrieben, die auf VCSEL-Laserdioden basieren, die durch einen Stromstoß bzw. einen Temperaturschock kurzzeitig bei einer Wellenlänge von ca. 1300nm durchstimmbar sind. Um bei der Vermessung des Auges Bewegungsartefakte auszuschließen, werden jedoch sehr hohe Sweep Rates (Wiederholrate) des Lasers im Bereich von 10-100kHz angegeben. Der offenbarte Entwurf für ein low-cost OCT System mit einer Wellenlänge von 1300nm ist jedoch für eine Ganzaugenmessung aufgrund der Absorption im Glaskörper ungeeignet. Weiterhin müsste bei der verwendeten Wellenlänge von 1300nm ein Sweep Range (Durchstimm-Bereich) von mindestens 25nm (besser 75nm) realisiert werden, um eine erforderliche Auflösung in Luft von 30μm

(besser 10μm) zu ermöglichen. Ein derartiger Sweep Range wird nicht in Aussicht gestellt werden. Die hier genannten Parameterkombinationen sind deshalb wenig geeignet, ein optisches Biometer aufbauen zu können, welches die Anforderungen an ein wettbewerbsfähiges System erfüllt.

In der DE 10 2008 028 312 A1 wird die Verwendung eines VCSEL-Laserdiode zur Augenvermessung beschrieben. Dabei wird die Laserdiode spektral schmalbandig bei einer Wellenlänge von ca. 850nm mit einer Kohärenzlänge von typisch 100mm und einer spektralen Breite ca. 0,007 nm betrieben und bietet damit eine ausreichende Scantiefe für die Vermessung der gesamten Augenlänge. Um bei einer maximalen spektralen Durchstimmung von 3nm bei 850nm Wellenlänge die erforderliche Messgenauigkeit erzielen zu können, ist jedoch von einer langsamen Durchstimmung dieser Laserdioden im Bereich <10Hz auszugehen. Damit ergibt sich hier die Notwendigkeit zur zusätzlichen Verwendung eines Positionserkennungssystems, welches bei langsam durchgestimmten Laserdioden erforderlich ist, um bewegte Objekte, wie das menschliche Auge, mit einer vergleichsweise langsamen Wiederholrate des durchgestimmten Lasers messen zu können.

In der WO 2018/119077 A1 wird ein miniaturisiertes, preiswertes OCT-System für ophthalmologische Anwendungen beschrieben. Insbesondere ist das System zur Messung der Dicke der Netzhaut vorgesehen, wobei das System aufgrund seiner Kompaktheit und Handlichkeit geeignet ist, dass ein Patient die Messungen zu Hause selbst vornehmen kann. Die hier beschriebenen SS-OCT-System basieren auf VCSEL-Laserdioden, die durch periodische Variation des Stromes gesteuert werden. Ein Ganzaugenscan ist mit den beschriebenen Systemen aufgrund der verwendeten Parameter nicht möglich. Stattdessen werden Systeme beschrieben, bei denen die Optik ein optisches Abtastelement beinhaltet, um zu ermöglichen, dass die Lichtquelle zu verschiedenen Orten auf der Netzhaut bewegt wird.

Literatur:

[1] Cole et. al.; "Ultra-Widely Tunable VCSELs", http://www.aomicro.com/tech/Cole_TUM_27_Sep_2012.pdf
[2] Nanoplus; "DFB laser diodes from 760 nm to 830 nm", https://nanoplus.com/fileadmin/user_upload/Data_sheets/nanoplus_DFB_760-830nm.pdf
[3] Philips, 760/763 nm single-mode VCSEL http://www.photonics.philips.com/pdf/ULM76X-SingleMode_TO5.pdf
[4] Moon et.al.; "VCSEL-based swept source for low-cost optical coherence tomography ", Biomedical Optics Express, Vol. 8, No. 2, Feb 1, 2017, p. 1110-1121
[5] Hogan B.; "Operation of VCSELs Under Pulsed Conditions", VIXAR Application Note, 21. Januar 2010
[6] Bublitz et al, "SS-OCT-Interferometry for measuring a sample; US 8,632,181 B2
[7] Choi W, et al, "Phase-sensitive swept-source optical coherence tomography imaging of the human retina with a vertical cavity surface-emitting laser light source"; Opt Lett. 2013;38(3):338-340
[8] Grulkowski et al: "Retinal, anterior segment and full eye imaging using ultrahigh speed swept source OCT with vertical-cavity surface emitting lasers", Biomedical Optics Express Bd. 3, Nr. 11, Nov. 1, 2012, p. 2733-2751

Der vorliegenden Erfindung liegt die Aufgabe zugrunde ein Verfahren zur Steuerung eines einfachen Halbleiterlaserdioden-basierten SS-Interferometer-Systems zu entwickeln, welches für Bildgebung und biometrische Messungen am Auge geeignet ist. Dabei sind die Parameter zur Steuerung der Laserstrahlquelle dahingehend zu optimieren, dass ein breiter Wellenlängenbereich, bei einer hohen Kohärenzlänge und einer vergleichsweise hohen Wiederholrate, durchstimmbar sind. Die biometrischen Messungen des Auges sollen dabei insbesondere mittels Ganzaugenscans erfolgen können.

Erfindungsgemäß wird die Aufgabe durch die Merkmale des unabhängigen Anspruchs gelöst. Bevorzugte Weiterbildungen und Ausgestaltungen sind Gegenstand der abhängigen Ansprüche.

Diese Aufgabe wird erfindungsgemäß mit dem Verfahren dadurch gelöst, dass mittels periodischer Strommodulation der Betrieb von einfachen Halbleiterlaserdioden so gestaltet wird, dass eine hochkohärente spektrale Laserlinie mit einer möglichst hohen Wiederholrate und über einen breiten Wellenlängenbereich durchstimmbar ist. Dabei werden die Parameter: Mittenwellenlänge, Sweep Rate, Sweep Range, optische Leistung am Auge und Kohärenzlänge so angepasst, dass das Verfahren für Bildgebung und biometrische Anwendungen durch Ganzaugenscans geeignet ist.

Vorteilhafte Ausgestaltungen betreffen die Steuerung der Halbleiterlaserdioden, insbesondere die periodische Strommodulation und/oder die Einstellung und Stabilisierung eines definierten nm/K-Gradienten, wobei ein Peltier-Element zum Einsatz kommen kann.

Bei dem Verfahren zur Steuerung eines Halbleiterlaserdioden-basierten SS-Interferometer-Systems, bei dem mittels periodischer Strommodulation der Betrieb von Halbleiterlaserdiode erfindungsgemäß so gestaltet wird, dass eine hochkohärente spektrale Laserlinie mit einer möglichst hohen Wiederholrate und über einen breiten Wellenlängenbereich durchstimmbar ist, sind Insbesondere dabei folgende Parameter vorgesehen:

- eine Mittenwellenlänge im Bereich von 600 - 1300nm,
- eine Sweep Rate von 10kHz oder 100kHz,
- eine Sweep Range im Bereich von 3 - 20nm und
- eine optische Leistung am Auge im Bereich von 50 - 20000μW,
- bei einer Kohärenzlänge von mindestens 20mm und
- Strompulse mit einer Dauer von 1μs bei einer Wiederholrate von 100kHz oder einer Dauer von 10μs bei einer Wiederholrate von 10kHz realisiert werden.

Bezüglich der optischen Leistung des Halbleiterlaserdioden-basierten SS-Interferometer-Systems ist vorgesehen, bei gegebener Wellenlänge möglichst die maximal zulässige optische Leistung am Patientenauge bereitzustellen, um bei Einhaltung der Sicherheitsbestimmungen ein maximales Signal/RauschVerhältnis realisieren zu können. Da ca. 50% der Leistung der Strahlquelle (Halbleiterlaserdiode) im optischen System des Interferometers als Verluste zu berücksichtigen sind, werden entsprechend höhere Leistungen der Halbleiterlaserdiode eingeplant:

| Mittenwellenlänge λ | maximale optische Leistung am Auge | Leistung der Halbleiterlaserdiode |
|---|---|---|
| 600nm | 390 μW | > 0,8 mW |
| 800nm | 620 μW | >1,2 mW |
| 1050nm | 1,95 mW | >4 mW |
| 1300nm | 15,6 mW | >31 mW |

Die optische Leistung bezieht sich auf die beispielsweise in der Lasernorm DIN EN 60825-1 definierte Strahlungsleistung, die am menschlichen Auge appliziert werden darf. Diese ist abhängig von der Wellenlänge und der zeitlichen Formung des Laserstrahles. Der Einfachheit halber beziehen sich die hier aufgeführten Laserleistungen auf cw-Betrieb der Diode und Laserklasse 1. Für gepulste Strahlung und andere Laserklassen können andere Impulsspitzenwerte zur Anwendung kommen. Gegebenenfalls müssen auch länderspezifische Normen berücksichtigt werden.

Das vorgeschlagene Halbleiterlaserdioden-basierte SS-Interferometer-System ist insbesondere für biometrische Messungen des Auges vorgesehen. Da die Darstellungen bevorzugt auf optischen, kohärenztomographischen Scan-Aufnahmen basieren, liegt die Hauptanwendung in der ophthalmologischen Diagnostik, Therapie und der Vorbereitung chirurgischer Eingriffe und deren Nachuntersuchung.

Die Erfindung wird nachfolgend anhand von Ausführungsbeispielen näher beschrieben. Hierbei werden die englischen und deutschen Begriffe synonym verwendet. Dazu zeigt

Figur 1:   eine Prinzipdarstellung zur Steuerung eines erfindungsgemäßen Halbleiterlaserdioden-basierten SS-Interferometer-Systems mittels periodischer Strommodulation und

Figur 2:   einige weitere optische Parameter der von der Halbleiterlaserdiode des SS-Interferometer-Systems emittierten Strahlung.

Das Halbleiterlaserdioden-basiertes SS-Interferometer-System besteht aus einer Halbleiterlaserdiode mit einer Steuereinheit, wobei die Steuereinheit ausgebildet ist, den Betrieb der Halbleiterlaserdiode mittels periodischer Strommodulation so zu steuern, dass eine hochkohärente spektrale Laserlinie in einer möglichst hohen Wiederholrate über einen breiten Wellenlängenbereich durchstimmbar ist.

Dazu ist die Steuereinheit ausgebildet zur periodischen Strommodulation Zeit und Amplitude der Stromstöße zu variieren.

Als Halbleiterlaserdioden kommen beispielsweise VCSEL-Laserdioden zum Einsatz, die auf einem GaAs-Wafer Oberflächenemitter oder einem single-mode AlGaInP-Wafer mit multi-quantum-well Struktur basieren können.

Insbesondere ist erfindungsgemäß vorgesehen nur single-mode VCSEL-Laserdioden einzusetzen. Multi-mode VCSEL-Laserdioden sind nicht vorgesehen.

Single-mode VCSEL Laserdioden zeichnen sich durch eine sehr geringe instantane Linienbreite, eine hohe Kohärenzlänge und die für biometrische Messungen erforderliche Messtiefe aus. Die single-mode VCSEL Laserdiode soll dabei eine Kohärenzlänge von mindestens 20mm, insbesondere aber 60 mm aufweisen. Damit wird gewährleistet, dass auch sehr lange Augen, wie sie bei einer hohen Myopie vorkommen, sicher vermessen werden können.

Somit eignet sich das System auch für Myopie-Reihenuntersuchungen, wie sie beispielsweise aktuell im asiatischen Raum durchgeführt werden, um eine Myopisierung der Bevölkerung einzudämmen.

Die single-mode VCSEL Laserdiode sollte mit einer spektralen Breite der Laserlinie von typischerweise 100 MHz eingesetzt werden. Entsprechende Laser sind z.B. bei der Firma Trumpf verfügbar.

Zur Einstellung und Stabilisierung eines definierten nm/K-Gradienten kann die VCSEL-Laserdiode in ein aktives Halbleitermaterial eingebettet sein, wobei das aktive Halbleitermaterial entsprechend dimensioniert und an die angrenzenden Halbleitermaterialschichten angepasst wird. Um die erzeugte Wärmesenke zu optimieren, kann wärmeleitfähiges Material und/oder ein Peltier-Element Verwendung finden.

Insbesondere kann das Design der VCSEL-Laserdiode so gestaltet sein, dass deren aktive Zone modifizierbar ist, um eine kontinuierliche optische Leistung am Auge von bis zu 20mW zu realisieren.

Erfindungsgemäß ist eine VCSEL-Laserdiode mit einer Wellenlänge im Bereich von 600 - 1300 nm, insbesondere von ca. 1050nm vorgesehen.

Dabei ist zu berücksichtigen, dass eine kürzere Wellenlänge einen geringeren Durchstimmbereich (engl.: Sweep Range) für eine gleiche Auflösung benötigt und insbesondere die Wiederholrate die größte technologische Herausforderung bei dieser Technologie ist. Demnach sind kurze Wellenlängen im roten Spektralbereich mit beispielsweise 600nm und im IR-Bereich mit beispielsweise 700nm dafür am besten geeignet. Beispielsweise ist eine single-mode AlGaInP Laserdiode mit einer multi-quantum-well Struktur und einer Wellenlänge von 690nm geeignet. Weiterhin sind VCSEL-Laserdioden mit einer Wellenlänge von 840nm verfügbar und gut geeignet.

Für eine Kataraktdurchdringung bzw. höhere Eindringtiefe in das Gewebe sind allerdings VCSEL-Laserdioden mit Wellenlänge von ca. 1050nm besser geeignet und deshalb zu bevorzugen.

Weiterhin ist erfindungsgemäß eine VCSEL-Laserdiode mit einer Wiederholrate (engl.: Sweep Rate) von 10kHz oder 100kHz vorgesehen. Dabei soll das erfindungsgemäße SS-Interferometersystem insbesondere in Abgrenzung zu [6] kein zusätzliches Bewegungssignal des Auges benötigen, um die Messergebnisse auswerten zu können.

Weiterhin ist zu berücksichtigen, dass eine Abhängigkeit der instantanen Linienbreite, der Kohärenzlänge und der Meßtiefe von der Durchstimmgeschwindigkeit und der Wiederholrate des A-scans besteht.

Erfindungsgemäß ist eine Optimierung dieser Abhängigkeit vorgesehen. Dabei steht eine ausreichende Meßtiefe von 60 mm für die Ganzaugen-Biometrie im Vordergrund.

Für die Durchstimmgeschwindigkeit/Wiederholrate des A-Scans wird mindestens eine Frequenz von 10 kHz gewählt, um auf jeden Fall Bewegungsartefakte des Auges ausschließen zu können. Eine Erhöhung auf 100 kHz ist für eine Ausgestaltung des biometrischen Messsystems vorgesehen, wenn die Messtiefe von 60 mm dadurch nicht unterschritten wird.

Eine weitere technische Herausforderung bei der Auswahl und Steuerung einer geeigneten VCSEL-Laserdiode ist in dem vorhandenen Temperaturgradienten der Wellenlängenänderung zu sehen. Für eine Durchstimmbarkeit von 5nm würde man dementsprechend eine Temperaturänderung von 50K benötigen.

Zur Realisierung großer Durchstimmbereiche bei einer Wiederholrate von ca. 10kHz müssen handelsübliche VCSEL-Laserdioden modifiziert werden oder sie sind gepulst zu betreiben. Dazu ist die vorhandene Steuereinheit ausgebildet, zur periodischen Strommodulation Zeit und Amplitude der Stromstöße zu variieren.

Hierzu ist erfindungsgemäß vorgesehen, dass die periodische Strommodulation (elektrisches Tuning) unabhängig von der Richtung der Wellenlängenänderung erfolgt, so dass sowohl up- als auch down-Sweep Verwendung finden.

Insbesondere ist vorgesehen, beim Durchstimmen der Wellenlänge mit Hilfe des Strompulses sowohl den up-sweep als auch den down-sweep der Wellenlänge für die Anwendung bei der erfindungsgemäßen einfachen swept source VCSEL Biometrie einzusetzen. Dabei wird mit steigendem Strom eine längere Wellenlänge eingestellt. Somit ist beim down-sweep zu beachten, dass sich in diesem Stromstoßintervall die Wellenlänge verkürzen wird.

Allgemein ist bekannt, dass ein Temperaturgradient von ca. 0,1nm/K realisierbar ist. Damit wäre für einen Durchstimmbarkeit von 5nm eine Temperaturänderung von 50K erforderlich.

Erfindungsgemäß ist vorgesehen diesen Gradienten auf > 0,1nm / K, vorzugsweise auf ca. 0,3 bis 0,5nm / K zu vergrößern.

Das wird dadurch erreicht, dass ein passendes aktives Halbleitermaterial ausgewählt wird. Es ist aber auch möglich, die VCSEL-Laserdiode zusätzlich in ein aktives Halbleitermaterial einzubetten.

VCSEL-Laserdioden für Wellenlängen zwischen 600nm und 1300nm basieren beispielsweise als Oberflächenemitter auf GaAs-Wafern.

Weiterhin ist die Verwendung von single-mode AlGaInP-Laserdioden mit einer multi-quantum-well Struktur bei einer Wellenlänge von 690nm und einem großen Temperaturgradienten von mindestens 0,25 nm/K vorgesehen.

Zur Optimierung der Wärmesenke zur Realisierung eines hohen Temperaturgradienten innerhalb der möglichen Betriebstemperatur der VCSEL-Laserdiode (z. B. -20 bis +70°C) ist beispielsweise die Ankopplung von hoch wärmeleitfähigem Material wie Kupfer, Indium, o. ä. an das Halbleitermaterial vorgesehen.

Neben der erfindungsgemäß rein elektrisch verursachten Wellenlängendurchstimmung der single-mode VCSEL Laserdioden ist außerdem auch eine Kombination der rein elektrischen mit einer zusätzlichen thermischen Durchstimmung der Wellenlänge vorgesehen.

Dazu wird das elektrisch gesteuerte Modul synchronisiert mit der Wärmesenke betrieben, welche die Umgebungstemperatur der single-mode VCSEL Laserdiode von prinzipiell - 80°C bis +180°C in einem gewissen Bereich von z.B. 100 K mit einer möglichst hohen Wiederholrate von > 10 kHz durchstimmt. Damit kann der sweep range (Durchstimmbereich der Wellenlänge in nm) erweitert und damit die Auflösung des Biometers gesteigert werden.

Zusätzlich kann eine aktive Kühlung mit Hilfe eines Peltier-Elementes erfolgen.

Da eine zusätzliche Kühlung jedoch dazu führen kann, dass sich Kondenswasser am Austrittsfenster der VCSEL-Laserdiode niederschlägt, ist erfindungsgemäß vorgesehen, das Austrittsfensters der VCSEL-Laserdiode mit einer Heizung zu versehen.

Wie bereits erwähnt sind für eine Kataraktdurchdringung bzw. höhere Eindringtiefe in das Gewebe VCSEL-Laserdioden mit Wellenlänge von ca. 1050nm besser geeignet.

Erfindungsgemäß ist dabei eine optische Leistung am Auge im Bereich von $50\mu W$ bis maximal $2000\mu W$, insbesondere größer $1000\mu W$ (bei einer Wellenlänge von 1050nm) vorgesehen.

Hierbei ist zu berücksichtigen, dass eine höhere optische Leistung nur im Rahmen der Lasersicherheitsbestimmungen realisiert werden dürfen. Zur Erreichung höherer optischer Leistungen sind Veränderungen im Design der VCSEL-Laserdioden erforderlich. Hierzu ist es beispielsweise möglich die aktive Zone oder andere Parameter der VCSEL-Laserdioden zu variieren, um eine optische Leistung von bis zu 2mW zu realisieren.

Erfindungsgemäß ist für den Aufbau eines VCSEL-basierten SS- Interferometer-Systems vorgesehen, dass die Einkopplung der von der Halbleiterlaserdiode abgegebene Laserstrahlung in das Interferometer mittels Freistrahl- oder Faseroptik erfolgt. Dazu ist es erforderlich, dass das Design der VCSEL-Laserdiode entsprechend gestaltet ist. In beiden Fällen ist die Strahlqualität bei gegebener Leistung ein wichtiges Kriterium, da diese die für die Ausführung der Messung nutzbare Laserleistung definiert.

Insbesondere bei einem Faser-basierten Design ist Wert darauf zu legen, dass eine möglichst verlustfreie Einkopplung erfolgt. Bei Monomode-Fasern mit einem Faserdurchmesser von $5\mu m$ kann eine nahezu verlustfreie Einkopplung mit Verlusten <10% bei einer Numerischen Apertur NA = 0,14 erreicht werden. Generell ist eine Einkopplung in Monomodefasern mit einem Kerndurchmesser von 3 bis 9 $\mu m$ vorgesehen.

Eine weitere Ausgestaltung der Erfindung betrifft die Erzeugung eines auf Wellenlänge geregelten Strompulses. Durch Auswertung der Ströme von zwei Fotodioden mit unterschiedlichen spektralen Verhalten, kann auf einfache Weise mit analoger Elektronik ein Messsignal der Wellenlänge erzeugt und als Feedback-System genutzt werden.

Beispielhaft wird das im Folgenden für den Wellenlängenbereich um 1050nm beschrieben.

In diesem Wellenlängenbereich beträgt die Änderung der spektralen Empfindlichkeit bei InGaAs-Fotodioden nur etwa +0,1A/W pro 100nm und bei Si-Fotodioden ca. -0,5A/W pro 100nm. Schwankungen der Leistung vom Laser wirken allerdings auf beide Fotodioden gleich aus. Deswegen bleibt nach geeigneter Normierung des Stromes der Si-Fotodiode mit dem Strom der InGaAs-Fotodiode nur eine Abhängigkeit des resultierenden Stroms von der Wellenlänge übrige, die ca. 0,4A pro 100nm ($4\mu A/nm$) beträgt. Dieses Messsignal kann nun verwendet werden, um den Strompuls so zu formen, dass der gewünschte Verlauf der Wellenlängenänderung erreicht wird.

Bevorzugt würde ein Verlauf der Wellenlänge über die Zeit erzeugt, der einer äquidistanten Änderung der Wellenzahl über der Zeit entspricht. Damit könnte die spektrale Information im Interferenzsignal (Frequenzraum) ohne weitere Entzerrung direkt durch Fouriertransformation zum Messergebnis im Ortsraum transformiert werden.

Eine geeignete Formung der Strompulse kann dadurch erfolgen, dass das wellenlängenabhängige Signal digitalisiert und algorithmisch verarbeitet wird (Verbesserung z.B. durch Filterung und Mittelung). Dann wird der Strompuls iterativ verformt bis das gewünschte Ergebnis erreicht ist.

Neben der geeigneten Formung der Strompulse bis zur Erreichung einer Linearität bzw. äquidistanten Änderung der Wellenzahl über der Zeit ist erfindungsgemäß weiterhin vorgesehen, strukturierte Strompulse einzusetzen, bei denen mehrfach der Strom unterbrochen wird, also ein schnell getakteter Strom die Einhüllende eines Strompulses ausfüllt. Dieser getaktete Strompuls wird erfindungsgemäß optimiert, bis seine Struktur in Abstand der Einzelpulse und Amplitude der Einzelpulse zu einer äquidistanten Änderung der Wellenzahl der single-mode VCSEL Laserdiode über der Zeit führt.

Eine weitere Möglichkeit wäre ein geschlossener analoger oder digitaler Regelkreis mit einem gewünschten Wellenlängenverlauf als Sollwert, dem wellenlängenabhängigen Signal als Messwert und dem Strompuls als Stellsignal.

Erfindungsgemäß ist weiterhin vorgesehen, die Formung von Strompulsen mit kontinuierlicher Änderung des Stroms innerhalb eines Impulses so zu optimieren, dass ein möglichst breiter sweep range (Durchstimmbereich der Wellenlänge in nm) der single-mode VCSEL Laserdiode erzeugt wird. So sind Bereiche > 3 nm bzw. insbesondere > 8nm bis hin zu 20 nm vorgesehen.

Neben der geeigneten Formung der Strompulse bis zur Erreichung eines möglichst breiten sweep range (Durchstimmbereich der Wellenlänge in nm) ist erfindungsgemäß weiterhin vorgesehen, strukturierte Strompulse einzusetzen, bei denen mehrfach der Strom unterbrochen wird, also ein schnell getakteter Strom die Einhüllende eines Strompulses ausfüllt. Dieser getaktete Strompuls wird erfindungsgemäß optimiert, bis seine Struktur in Abstand der Einzelpulse und Amplitude der Einzelpulse zu einem maximalen sweep range bis hin zu ca. 20 nm (Durchstimmbereich der Wellenlänge in nm) der single-mode VCSEL Laserdiode über der Zeit führt.

In einer letzten Ausgestaltung der Erfindung wird der kurze Strompuls in einem Bereich oberhalb des

Schwellstromes $I_{th}$, mit einer Dauer der Stromanstiegsflanke von < 500 μs, insbesondere < 50 μs bis hin zu ≈ 1 μs gewählt. Damit wird zusätzlich zum beschriebenen thermisch induzierten, spektralen Sweep Range, der z.B. 0,1 nm/K beträgt, eine nicht thermisch induzierte Erweiterung des spektralen Sweep Range (athermischer, spektraler Sweep Range) erreicht. Der erfindungsgemäß genutzte Effekt beruht auf der rein elektrisch induzierten Verschiebung der elektronischen Niveaus/Bandlücke des Halbleiter-Lasermaterials aufgrund der kurzzeitig beaufschlagten hohen elektrischen Feldstärken. Dieser erweiterte Anteil des spektralen Sweep Range wird insbesondere durch die Art des Strompulses und dessen Anstiegsflanken erzeugt. Vorteilhaft bei dieser Betriebsart ist der geringe oder vernachlässigbare Aufwand bzgl. der thermischen Stabilität des SS-OCT-Systems. Kühlung und thermische Stabilisierung kann bei dieser Betriebsart minimiert oder vermieden werden.

Diese erfindungsgemäße Betriebsart ist eingestellt, wenn die Bedingung

$$\Delta\lambda > \Delta T \; \Delta\lambda_{therm}$$

gilt, wobei

$\Delta\lambda$        - der spektralen Sweep Range,
$\Delta\lambda_{therm}$    - der thermisch induzierten spektralen Sweep Range (in nm/K) und
$\Delta T$        - der Temperaturänderung während des Stromimpulses (in K) entsprechen.

Messtechnisch kann diese Betriebsart z.B. mit einem integrierten Temperatursensor für $\Delta T$ am Laserchip und einer Messung während des Strompulses in Kombination mit der Vermessung des erreichten spektralen Sweep Bereichs $\Delta\lambda$ bei bekanntem, stationär vermessenen spektralen Driftverhalten des Lasermaterials $\Delta\lambda_{therm}$ erfolgen.

Bei dem vorgeschlagenen Verfahren zur Steuerung eines Halbleiterlaserdioden-basierten SS-Interferometer-Systems wird der Betrieb der Halbleiterlaserdioden mittels periodischer Strommodulation so gestaltet, dass eine hochkohärente spektrale Laserlinie mit einer möglichst hohen Wiederholrate und über einen breiten Wellenlängenbereich durchstimmbar ist.

Erfindungsgemäßen sind dabei folgende Parameter vorgesehen:

- eine Mittenwellenlänge im Bereich von 600 - 1300nm,
- eine Sweep Rate von 10kHz oder 100kHz,
- eine Sweep Range im Bereich von 3 - 20nm und
- eine optische Leistung am Auge im Bereich von 50 - 20000 μW,
- bei einer Kohärenzlänge von mindestens 20mm und
- Strompulse mit einer Dauer von 1 μs bei einer Wiederholrate von 100kHz oder einer Dauer von 10 μs bei einer Wiederholrate von 10kHz realisiert werden..

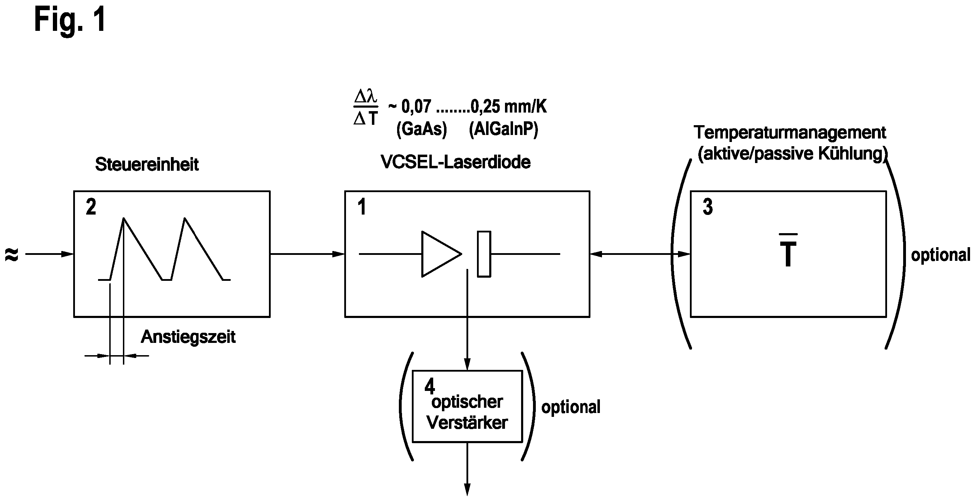

Hierzu zeigt die **Figur 1** eine Prinzipdarstellung der Steuerung eines erfindungsgemäßen Halbleiterlaserdioden-basierten SS-Interferometer-Systems mittels periodischer Strommodulation.

Das Halbleiterlaserdioden-basierte SS-Interferometer-System besteht aus einer Halbleiterlaserdiode **1** mit einer Steuereinheit **2,** wobei die Steuereinheit **2** ausgebildet ist, den Betrieb der Halbleiterlaserdiode **1** mittels periodischer Strommodulation so zu steuern, dass eine hochkohärente spektrale Laserlinie in einer möglichst hohen Wiederholrate über einen breiten Wellenlängenbereich durchstimmbar ist.

Dazu ist die Steuereinheit **2** ausgebildet zur periodischen Strommodulation Zeit und Amplitude der Stromstöße zu variieren. Insbesondere ist die Steuereinheit **2** ausgebildet, kurze Anstiegszeiten der Strompulse zum elektrischen Pumpen der Halbleiterdiode **1** abzugeben.

Dabei ist die Halbleiterlaserdiode **1** insbesondere eine VCSEL-Laserdiode mit einer Variation der Wellenlänge der hochkohärenten spektralen Laserlinie in Abhängigkeit von der aktuellen Betriebstemperatur. So ist für eine Halbleiterlaserdiode auf der Basis von GaAs beispielsweise eine Variation $\Delta\lambda_{therm}/\Delta T$ von 0,07 nm/K und für eine Halbleiterlaserdiode auf der Basis von ALGaInP von 0,25 nm/K vorgesehen.

Zusätzlich ist anzumerken, dass erfindungsgemäß infolge der kurzen Anstiegszeit der Stromstöße von der Steuereinheit **2** ein breiterer Durchstimmbereich der Wellenlänge $\Delta\lambda$ über den durch die thermische Variation bekannten Wert hinaus erreicht werden kann ($\Delta\lambda > \Delta T \; \Delta\lambda_{therm}$).

Optional ist eine primär für den stabilen Langzeitbetrieb der SS-Laserquelle Temperaturstabilisierung bzw. ein Temperaturmanagement durch eine Wärmesenke **3** welches aktiv als auch passiv arbeiten kann, vorgesehen. Wenn die Wärmekapazität der Halbleiterlaserdiode **1** und Ihre bereits vorhandene Wärmesenke für einen stabilen Betrieb ausreicht, kann auch auf diese zusätzlichen Merkmale der Wärmesenke **3** verzichtet werden.

Um diese Wärmesenke **3** zu optimieren, kann wärmeleitfähiges Material und/oder ein Peltier-Element Verwendung finden. Eine aktive Abkühlung des Laserchips der Halbleiterlaserdiode **1** ist synchron zur periodischen Strommodulation durch die Steuereinheit **2** nur in den Zeiten außerhalb der Stromanstiegsflanke (Rise Time) vorgesehen.

Neben der Funktion der Abkühlung des Laserchips infolge der Aufheizung durch den Strompuls der Steuereinheit **2** für einen thermisch stabilen Betrieb ist die Wärmesenke **3** optional auch für eine zusätzliche Heizung des Laserchips der Halbleiterlaserdiode **1** synchron zum Strompuls der Steuereinheit **2** nur innerhalb der Stromanstiegsflanke vorgesehen. Dadurch ist es möglich die thermisch induzierte Wellenlängenverstimmung durch eine Erhöhung der Temperaturänderung $\Delta T$ nicht nur auf Basis der elektrisch induzierten Temperaturerhöhung, sondern zusätzlich noch auf Basis dieser optionalen direkten aktiven Temperaturerhöhung innerhalb der Zeit der Stromanstiegsflanke zu erhöhen.

Von der Halbleiterlaserdiode **1** wird vorzugsweise eine Ausgangsleistung im Bereich einiger mW mit den gewünschten Parametern eines SS-Interferometer-Systems für OCT- und biometrische Anwendungen abgegeben, die Verluste im weiteren optischen System ausgleichen kann und ein gutes Signal-Rausch-Verhältnis unter Beachtung der maximalen ophthalmologisch einsetzbaren Intensitäten in der diagnostischen Anwendung erreicht. Ist die Ausgangsleistung der Halbleiterlaserdiode **1** zu gering wird optional ein Halbleiter basierter optischer Verstärker (engl.: Semiconductor Optical Amplifier, kurz: SOA) als SOA **4** in den optischen Strahlengang integriert.

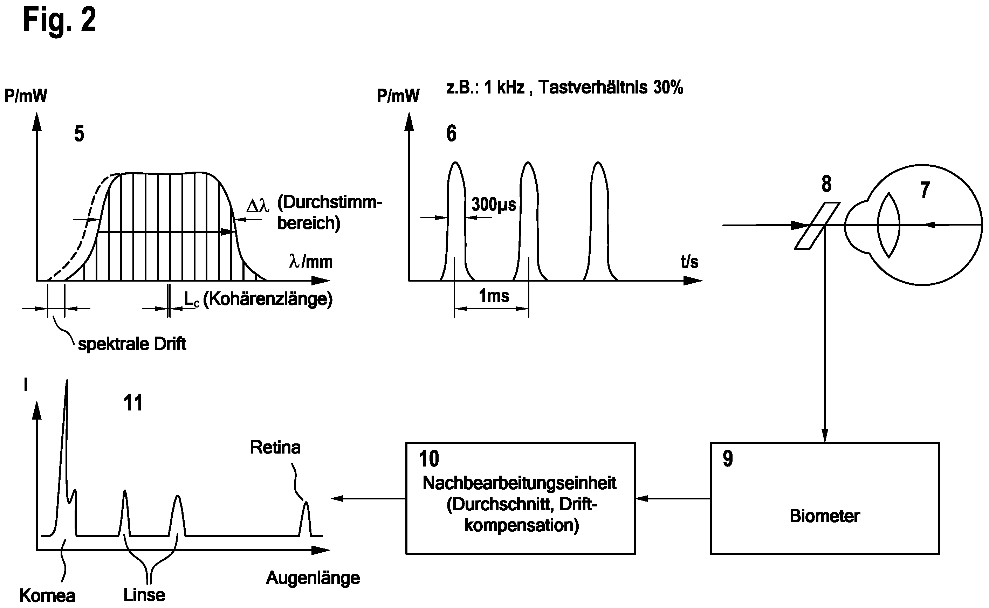

In der **Figur 2** sind einige weitere optische Parameter der von der Halbleiterlaserdiode des SS-Interferometer-Systems emittierten Strahlung dargestellt.

Das Diagramm **5** skizziert die Durchstimmung der einzelnen hochkohärenten Wellenlängen über einen Wellenlängenbereich $\Delta\lambda$, den sogenannten Sweep Range mit einer Leistung **P** im mW-Bereich. Die spektrale Auflösung des Sweeps bestimmt die Kohärenzlänge Lc bzw. die Meßtiefe in Luft wie in der Grafik angegeben. Diese Funktionalität wird innerhalb einer Stromanstiegsflanke (Rise Time) der erfindungsgemäßen Halbleiterlaserdiode 1 realisiert. In dieser Darstellung ist eine Verschiebung (Spectral Jitter) des Durchstimmbereiches $\Delta\lambda$ dargestellt, welche insbesondere durch eine Erwärmung der Halbleiterlaserdiode **1** nach längerem Betrieb entstehen kann. Dieser Effekt kann durch ein Temperaturmanagement mittels der Wärmesenke **3** unterdrückt werden.

Das Diagramm **6** skizziert mit einem Beispiel die zeitlichen Emissionsparameter mit einzelnen Laserpulsen, bei denen jeder Laserpuls von $300\mu s$ die spektrale Durchstimmcharakteristik der hochkohärenten Wellenlänge innerhalb $\Delta\lambda$ nach Diagramm **5** enthält. Hier werden diese Laserpulse mit einer Repetitionsrate von 1kHz und damit einem Pulsabstand von 1ms gezeigt. Dabei wird ein Duty Cycle von 30% realisiert. So hat der Laserchip beispielsweise nach einem aktiven Strompuls von $300\mu s$ wieder $700\mu s$ Zeit zur thermischen und/oder elektromagnetischen Relaxation für eine langzeitstabile Funktionalität.

Die Laserstrahlung der Halbleiterlaserdiode **1** mit den Eigenschaften nach den Diagrammen **5** und **6** wird innerhalb eines nicht detailliert dargestellten interferometrischen Systems in das zu untersuchende Auge **7** eingekoppelt. Die aus dem Auge rückgestreute Lichtintensität wird mittels Strahlteiler **8** innerhalb eines Biometers **9** detektiert. Die Rohsignale des Biometers **9** werden mit Hilfe einer Post Processing Einheit **10** verarbeitet, um einen Tiefenscan über das gesamte Auge **7** (auch A-Scan genannt), insbesondere mit Informationen zur Lage und zu den Abständen der Kornea, der Linse und der Retina als Messergebnis **11** auszugeben. In einem hier nicht skizzierten Vorgehen kann dieser A-Scan lateral über die Pupillenöffnung des Auges gescannt werden, um als sogenannter B-Scan ein Schnittbild der genannten Abstände im Auge **7** zu erzeugen.

Um die durch thermische Umgebungseinflüsse und Änderungen der Betriebstemperatur entstehende unerwünschte Drift des Durchstimmbereiches der Wellenlänge (sweep range), also den sogenannten Spectral Jitter zu kompensieren, kann die single-mode VCSEL Laserdiode auf eine temperierte Wärmesenke im Temperaturbereich von prinzipiell -80° bis +180° C mit stabilisierter Temperatur bei einer Temperaturkonstanz von ca. +/- 1 K eingebaut werden. Dabei können außerdem bei niedrigen Temperaturen durch hohe Ströme auch hohe Ausgangsleistungen der Monomode VCSEL Laserdiode erzielt werden.

Um die Wellenlängendrift in Abhängigkeit von der Temperatur ohne eine zusätzliche Wärmesenke zu realisieren, ist erfindungsgemäß der Einsatz eines faseroptischen Bragg-Gitters im Interferometer des kurzkohärenten biometrischen Messsystems vorgesehen, welches eine Wellenlängenreferenz vorgibt, mit der das Messsystem kontinuierlich rekalibriert werden kann.

Zur besseren spektralen Stabilisierung des Durchstimmbereichs kann ein (z. B. faserbasierter) Bragg-Reflektor/Gitter (FBG) verwendet werden. Dieser Reflektor wird so im System eingebracht, dass während eines Durchstimmvorgangs ein Lichtreflex bei einer exakt definierten Wellenzahl entsteht. Dieser Lichtreflex kann mit dem Interferenzsignal gemeinsam aufgenommen werden. Er dient als Referenz zur genauen und absoluten spektralen Lokalisierung des spektralen Interferenzsignals.

Hierzu beschreibt W. Choi u. a. in [7] eine Lösung, bei der einer der Detektionsarme zum Balanced Detektor über ein FBG führt. Das FBG führt im Detektionssignal zu einem "Notch", welches für die spektrale Referenzierung der Signale verwendet werden kann.

Eine erste Variante des Verfahrens betrifft die Bildgebung basierend auf einem SS-OCT-System. Auch hier soll eine hochkohärente spektrale Laserlinie mit einer möglichst hohen Wiederholrate und über einen breiten Wellenlängenbereich durchstimmbar sein.

Für die Vermessung des vorderen Augenabschnittes bis zur Rückseite der Linse würde beispielsweise eine Messtiefe in Luft bzw. Kohärenzlänge Lc von ca. 25mm genügen.

Mit einer Messanordnung zur Bestimmung der biometrischen Werte werden im Wesentlichen aus sogenannten A-Scans oder B-Scans Messwerte wie Augenläng AL, Linsendicke LD, Vorderkammertiefe VKT und die Hornhautdicke HHD, beispielsweise zum Zweck der IOL-Berechnung bestimmt. Hierfür ist eine axiale Auflösung von ca. 100$\mu$m in Luft ausreichend.

Beispielhaft wird dafür auf den IOLMaster 500 der Zeiss Meditec AG verwiesen, in dem eine Multimode-Laserdiode mit einer Halbwertsbreite von $\Delta\lambda \approx 2{,}7$nm bei einer Mittenwellenlänge von 785nm eine Auflösung von 100$\mu$m in Luft realisiert.

Die axiale Auflösung $\Delta$**z** bei Gauss'scher spektraler Leistungsdichte ist definiert als:

$$\Delta z = \frac{2ln2}{\pi} * \frac{\lambda^2}{\Delta\lambda}$$

in der:

$\Delta\lambda$     die Halbwertsbreite und
$\lambda$     die Mittenwellenlänge definieren.

Auch andere typische spektrale Leistungsdichten können durch die obige Formel gut approximiert werden. Bei einer Halbleiterlaserdioden mit 1050nm Mittenwellenlänge ist ein $\Delta\lambda \approx 5$nm nötig, um die Auflösung von 100$\mu$m in Luft zu realisieren.

Benutzt man eine Halbleiterlaserdioden bei 840nm zentraler Wellenlänge und 5nm elektrischen Durchstimmbereich erhält man einen noch besseren Wert von ca. 65$\mu$m Auflösung in Luft. Bei dieser Wellenlänge von 840nm erreicht man die für die Biometrie erforderliche axiale Auflösung von 100$\mu$m in Luft bereits bei einem Durchstimmbereich von ca. 3nm.

Dementsprechend ergeben sich daraus die folgenden Werte:

| Mittenwellenlänge $\lambda$ | Halbwertsbreite $\Delta\lambda$ | Auflösung in Luft $\Delta$z |
|---|---|---|
| 600nm | 16nm | 10$\mu$m |
| 600nm | 5nm | 30$\mu$m |
| 600nm | 3.5nm | 50$\mu$m |
| 800nm | 28nm | 10$\mu$m |
| 800nm | 10nm | 30$\mu$m |
| 800nm | 6nm | 50$\mu$m |
| 1050nm | 50nm | 10$\mu$m |
| 1050nm | 16nm | 30$\mu$m |
| 1050nm | 10$\mu$m | 50$\mu$m |
| 1300nm | 75nm | 10$\mu$m |
| 1300nm | 25nm | 30$\mu$m |
| 1300nm | 15$\mu$m | 50$\mu$m |

Erfindungsgemäß ist eine Halbwertsbreite (was dem Sweep Range der Halbleiterlaserdioden entspricht) im Bereich von 10 - 100nm, insbesondere von mindestens 16nm bei 1050nm vorgesehen.

Weiterhin ist erfindungsgemäß eine VCSEL-Laserdiode mit einer Wiederholrate (engl.: Sweep Rate) von 10kHz oder 100kHz vorgesehen.

In diesem Zusammenhang wird noch einmal auf den bereits erwähnten Zusammenhang zwischen Bewegungsartefakten, Wiederholrate und Signal-Rausch-Verhältnis verweisen.

Die Sensitivität des SS-OCT-Systems skaliert mit der im Gewebe deponierten Energie (Anzahl der Photonen) und nicht mit der Peak-Leistung. Deshalb wird erfindungsgemäß angestrebt, die Sweep Rate zum Ausschluss von Bewegungsartefakten des Auges schnell genug, aber auch ausreichend lang zu wählen, um bei gegebener Leistung der Laserdiode eine ausreichend große Pulsenergie zur Verfügung stellen zu können.

Erfindungsgemäß ist dabei eine optische Leistung im Bereich von 50 μW bis maximal 20000μW,insbesondere maximal 2000μW (bei einer Wellenlänge von 1050nm) vorgesehen.

Auch hier wird noch einmal auf die Einhaltung der bereits erwähnten Lasersicherheitsbestimmungen verweisen.

Es ist aber auch möglich, andere Parameter zu variieren. So kann beispielsweise die Linienbreite der VCSEL-Laserdiode verbreitert werden.

Erfindungsgemäß ist weiterhin eine Kohärenzlänge Lc von mindestens 60mm vorgesehen, da die daraus resultierende Messtiefe zur Vermessung des gesamten menschlichen Auges nötig ist.

Es kann jedoch auch ein SS-OCT System mit geringer axialer Auflösung >30μm, insbesondere >50μm realisiert werden. Dies ist zum Beispiel von Interesse für die tomografische und volumetrische Darstellung des gesamten Auges (Vorderkammer bis zum Augenhintergrund). Ebenfalls kann der Verlust in axialer Auflösung durch "machine-learning"-Algorithmen kompensiert werden. Hierfür müssen zuerst Bilder mit Standardauflösung aufgenommen werden, und diese Bilder zum Training der Algorithmen verwendet werden. Die machinelearning basierenden Algorithmen lernen dann die Auflösung auch in den Bildern mit niedriger axialer Auflösung zu verbessern.

Zur Erweiterung des Anwendungsbereiches der erfindungsgemäßen Technologie ist insbesondere eine verbesserte Auswertung der Ganzaugenscans als A-Scan und/oder als B-scan mit Hilfe von Algorithmen auf Basis des Machine Learning / Deep Learning / Artificial Intelligence vorgesehen. Dabei ist der Vorteil des erfindungsgemäßen Biometers die hohe Messtiefe, welche Ganzaugenscans mit einer hohen Repetitionsrate von z.B. 28 kHz A-Scanrate erlaubt. Nachteilig ist jedoch, dass die Auflösung auf Grund der geringen Sweep-Range von ca. 5 nm gering ist im Vergleich zu modernen hochauflösenden ss-OCT Systemen mit bis zu 100 nm Sweep-Range. Daher ist vorgesehen, mindestens eine Anzahl von ca. 20 Patientenaugen sowohl mit dem erfindungsgemäßen Biometer und gleichzeitig mit einem anderen kommerziell verfügbaren ultrahoch auflösenden OCT System ganz und/oder teilweise zu vermessen. Mittels Algorithmen auf Basis des Machine Learning / Deep Learning / Artificial Intelligence sollen nun die bekannten hochaufgelösten Messungen mit den Messungen des erfindungsgemäßen Biometers verglichen und bewertet werden. Als Ergebnis sollen diese Algorithmen spätere Einzelmessungen des Biometers im A- und/oder B-Scan in Ihrer Auflösung und Genauigkeit verbessern.

Einer zweiten Variante des Verfahrens zur Steuerung eines Halbleiterlaserdioden-basierten SS-Interferometer-Systems, betrifft biometrische Anwendungen, insbesondere durch Ganzaugenscans.

Bei dem vorgeschlagenen Verfahren zur Steuerung eines Halbleiterlaserdioden-basierten SS-Interferometer-Systems wird mittels periodischer Strommodulation der Betrieb von Halbleiterlaserdioden so gestaltet, dass eine hochkohärente spektrale Laserlinie mit einer möglichst hohen Wiederholrate und über einen breiten Wellenlängenbereich durchstimmbar ist. Erfindungsgemäß sind dabei für biometrische Anwendungen folgende Parameter vorgesehen:

- eine Mittenwellenlänge im Bereich von 600 - 1300nm,
- eine Sweep Rate von 10kHz oder 100kHz,
- eine Sweep Range im Bereich von 3 - 20nm und
- eine optische Leistung am Auge im Bereich von 50 - 20000μW,
- bei einer Kohärenzlänge von mindestens 20mm und
- Strompulse mit einer Dauer von 1μs bei einer Wiederholrate von 100kHz oder einer Dauer von 10μs bei einer Wiederholrate von 10kHz realisiert werden.

Die Halbleiterlaserdioden werden mittels periodischer Strommodulation betrieben, indem Dauer und Amplitude und Form (Anstiegsflanke) der Stromstöße variiert werden.

Für eine maximale Wiederholrate ist es erforderlich, einen maximalen Temperaturgradienten durch einen Stromstoß innerhalb der aktiven VCSEL-Halbleiterschicht zu erzielen. Um jedoch bei einer nahezu kontinuierlichen Betriebsweise eine hohe Wiederholrate bei gleichbleibenden Laserparametern erzielen zu können, ist eine gleiche mittlere Temperatur des aktiven Materials sicher zu stellen.

Das gelingt bei einer lediglich passiven Kühlung der VCSEL-Laserdioden erfindungsgemäß durch:

- einen kurzen Strompuls in einem Bereich oberhalb des Schwellstromes $I_{th}$, wobei insbesondere die Dauer der Stromanstiegsflanke < 500μs bis hin zu ≈ 50ns beträgt, und
- einem Tastverhältnis (engl.: duty cycle) welches ausreichend ist, um eine Abkühlung der aktiven VCSEL-Halbleiterschicht auf eine stabile Betriebstemperatur im Bereich von +/- 2 K erlaubt. Insbesondere ist dafür erfindungsgemäß ein Tastverhältnis von < 50% bis zu 1%, vorzugsweise 30% vorgesehen. So kann beispielsweise bei einem Strompuls von 100ns nach 10.000ns bzw. 10μs ein nächster Strompuls erfolgen und eine Wiederholrate von 100kHz eingestellt werden.

Einer vorteilhaften Ausgestaltung entsprechend kann ein erfindungsgemäßes VCSEL-basiertes SS-Interferometer-System durch folgende technische Parameter charakterisiert werden:

- einer Sweep Rate von 28 kHz,
- einem Duty Cycle von 30%,
- bei einer mittleren Laserwellenlänge von 840nm,
- einer Sweep Range von 5nm,
- einer Ausgangsleistung der VCSEL-Laserdiode von 2mW,
- was einer Leistung am Auge von 0,2mW und damit einer Messempfindlichkeit von 100dB entspricht.

Insbesondere ist bei dieser Optimierung vorgesehen, dass eine höhere Peak-Leistung erreicht werden kann, wenn ein kürzerer Treiberstrompuls verwendet wird, so dass ein Tastverhältnis von <10% realisierbar ist. Allerdings wird dann auch die im Gewebe deponierte Energie (Anzahl von Photonen) um einen Faktor >10 kleiner.

Erfindungsgemäß ist eine Halbwertsbreite (was dem Sweep Range der Halbleiterlaserdioden entspricht) im Bereich von 3 - 20nm, insbesondere von ca. 5nm bei 1050nm für biometrische Anwendung vorgesehen.

Erfindungsgemäß wird weiterhin zur Erzielung einer stabilen stationären Betriebstemperatur des VCSEL-basierten SS-Interferometer-Systems ein bezüglich der Messempfindlichkeit und der thermischen Stabilität angepasstes Tastverhältnis im Bereich bis von 1% bis 50%, insbesondere von 30% realisiert.

Da erfindungsgemäß eine minimale Wiederholrate von 1kHz vorgesehen ist, wäre bei Strompulsen von 100ns Dauer auch ein Tastverhältnis von 0,01% möglich, was eine 10000-mal längere Abkühlzeit gegenüber der Strompulszeit im aktiven Lasermaterial bedeutet. Um den Aufwand für die erforderliche Steuerung in Grenzen zu halten, ist ein Tastverhältnis von 0,1% realistisch. Dadurch lässt sich eine stabile, stationäre Betriebstemperatur des SS-OCT-Systems einstellen. Im Rahmen der bereits oben beschriebenen Limitationen der für die Messung erforderlichen Pulsenergie ist ein Tastverhältnis von 10% angestrebt und bei einem Tastverhältnis von 30% eine erste praktische Lösung gefunden worden. Da selbst bei einem Tastverhältnis von 30% der spektrale Durchstimmbereich dauerhaft stabil bleibt und keine spektrale Drift erfolgt ist bei der erfindungsgemäß gewählten Konfiguration für eine gute Wärmeableitung für das aktive Volumen der Laserdiode gesorgt worden.

In oben genannter vorteilhafter Ausgestaltung konnte eine mittlere Leistung der Diodenemission von 2mW gesichert werden. Bei der Anwendung dieser durchstimmbaren VCSEL-Laserdiode kann vom VCSEL-basierten SS-Interferometer-System eine Messleistung am Auge von 200µW bereitgestellt und eine biometrische Vermessung des Auges mit einer Empfindlichkeit von 100dB erzielt werden.

Erfindungsgemäß ist weiterhin vorgesehen den Stromstoß zum Pumpen der Laserquelle zeitlich derart in seiner Verlaufsamplitude zu steuern, dass man innerhalb des spektralen Durchstimmbereiches der Laseremission einen über die Zeit des Stromstoßes linearen Verlauf der emittierten Wellenlängen erhält. Dies ist vorteilhaft um Fehlerquellen in der Auswertung der Messsignale zu minimieren.

Wie bereits erwähnt, skaliert die Sensitivität des VCSEL-basierten SS-Interferometer-Systems mit der im Gewebe deponierten Energie (Anzahl der Photonen) und nicht mit der Peak-Leistung. Deshalb ist erfindungsgemäß eine vorteilhafte Optimierung vorgesehen, bei der die Laserdiode im Pulsbetrieb eine >10-fache Leistung erlaubt, was prinzipiell noch als praktisch machbar angesehen wird.

Letztendlich beschränkt die thermische Belastung die Ausgangsleistung der Laserdiode. Deshalb ist ein optimaler Durchstimmbereich insbesondere bei niedrigen Betriebstemperaturen vorgesehen.

Dem entsprechend ist für den erfindungsgemäßen Betrieb des VCSEL-basierten SS-Interferometer-Systems vorgesehen, für die VCSEL-Laserdiode einen stabilen nm/K-Gradient zu definieren und einzustellen.

Wie bereits erwähnt, ist ein Temperaturgradient von ca. 0,1nm/K realisierbar und damit für einen Durchstimmbereich von 5nm eine Temperaturänderung von 50K erforderlich. Erfindungsgemäß ist vorgesehen diesen Gradienten auf >0,1nm / K, vorzugsweise auf ca. 0,3 bis 0,5nm / K einzustellen. Dies kann unter anderem durch die zuvor genannten Maßnahmen erreicht werden.

Erfindungsgemäß ist dabei eine optische Leistung im Bereich von 50 µW bis maximal 20000µW,insbesondere maximal 2000µW (bei einer Wellenlänge von 1050nm) vorgesehen.

Deshalb ist erfindungsgemäß vorgesehen den Treiberstrom der Laserdiode in einem Bereich oberhalb des Schwellstromes $I_{th}$ durchzustimmen, wobei der minimale und der maximale Stromwert dieses Bereiches eine Ausgangsleistung der Laserdiode für die Messung am Auge in der Korneaebene von mindestens 50µW ergibt.

Einer weiteren vorteilhaften Ausgestaltung entsprechend sind für die periodische Strommodulation Taktschwankungen von < 1ns, eine Wiederholbarkeit der Strompulse < 10ns und eine Amplitudenstabilität von < +/-5% einzustellen und zu gewährleisten.

Deshalb ist zur Optimierung der bereits angesprochenen Wiederholbarkeit des Durchstimmbereichs vorgesehen:

- eine sehr geringe Taktschwankung bei den Steuerpulsen im Bereich von <1ns einzustellen,
- die Wiederholbarkeit der Steuerpulse in ihrer Abfolge < 10ns zu halten und
- in Ihrer Amplitudenstabilität im zeitlichen Verlauf < +/- 5% einzustellen.

Dadurch wird gewährleistet, dass der Wellenlängenbereich des Tunings gleichbleibt. Eine Änderung des Wellenlängenbereichs > 0,1nm würde sowohl die axiale Auflösung, als auch die axiale Skalierung und damit die Genauigkeit der biometrischen Messungen beeinflussen.

Weiterhin ist insbesondere bei einer mangelhaften Erfüllung der genannten erforderlichen Stabilitätsanforderungen an den spektralen Durchstimmbereich vorgesehen, die Variation der Trigger- und Treibersignale durch ein mechanisch stabiles Referenzsignal des Interferometers zu korrigieren. Die Korrektur kann zum Beispiel durch Korrelation und Registrierung der spektralen Interferenzmuster des Referenzsignals erfolgen.

Die erfindungsgemäße Lösung zum Aufbau eines einfachen durchstimmbaren Diodenlasers beruht auf der Tatsache, dass bei einer Temperaturänderung im aktiven Lasermaterial eine Wellenlängenänderung erzielbar ist. Dabei kann diese Temperaturänderung indirekt durch den elektrischen Strompuls oder/und durch eine zusätzliche Heizung bzw. Abkühlung erfolgen.

Zusätzlich zu diesem erfindungsgemäß gewünschten Effekt ist jedoch mit der Temperaturänderung des aktiven Materials der Laserdiode auch eine Änderung der Ausgangsleistung bei gegebenem Pumpstrom gegeben. Deshalb ist zunächst erfindungsgemäß die Wahl eines Designs für die Laserdiode vorgesehen, bei dem die Änderung der Ausgangsleistung in Abhängigkeit von der Betriebstemperatur gering bzw. reproduzierbar ist. Weiterhin ist bezüglich dieser Abhängigkeit eine Optimierung der zuvor genannten Betriebsbedingungen vorgesehen.

Neben den bereits genannten technischen Herausforderungen sind jedoch noch weitere Abhängigkeiten zu berücksichtigen, die auf die Auswahl und Steuerung geeigneter VCSEL-Laserdioden Einfluss haben.

Die Anwendung der erfindungsgemäßen Messtechnik am bewegten Auge erfordert schnelle Messzeiten, um Bewegungsartefakte im Messsignal ausschließen zu können. Die Messzeiten sollten für eine Einzelmessung ca. </= 1ms betragen. Demzufolge ist eine Kombination von Duty Cycle und Sweep Rate von beispielsweise 10% und 100Hz zu wählen. Bei dieser zeitlichen Durchstimmrate ist dann ebenfalls die spektrale Durchstimmbreite im Bereich von 3-20nm erforderlich, um die erforderliche Auflösung sicher zu stellen. Das bekannte Verhalten der Laserdioden ist jedoch, dass man insbesondere bei langsamen Durchstimmraten eine hohe Durchstimmbreite erzielt und damit eine weitere Herausforderung für die Realisierung des erfindungsgemäßen Messsystems. Für diese kombinierte Anforderung ist die Auswahl einer auf Grund des Designs der Laserdioden optimalen Version vorgesehen.

Weiterhin ist für eine reproduzierbare Auswertbarkeit der Messsignale eine absolut konstante spektrale Durchstimmbreite erforderlich. Andererseits ist auf Grund der im vorgesehenen Messbetrieb erforderlichen, wiederholten elektrischen/thermischen Durchstimmung der Laserdiode eine Temperaturdrift und damit eine Wellenlängendrift des Durchstimmbereichs die Folge.

Für diese Anforderung ist die Auswahl einer optimalen Version einer Laserdiode vorgesehen, die z.B. eine hohe Wärmeleitfähigkeit oder eine hohe Wärmekapazität und damit eine geringe unerwünschte Temperaturdrift besitzt.

Ein großer Sweep Range bedingt die Einstellung einer hohen Temperatur einer VCSEL-Laserdiode. Bei hohen Temperaturen ist jedoch ein Abfall der optischen Leistung der Laserdiode vorhanden.

Weiterhin hängt das genannte dynamische thermische Verhalten der VCSEL-Laserdioden von der Betriebstemperatur an sich ab. So erhält man bei höherer Betriebstemperatur:

- eine höhere Laserschwelle und
- eine niedrigere maximale Leistung $P_{max}$ als auch einen niedrigeren maximalen Strom $I_{max}$

Folglich ist erfindungsgemäß vorgesehen, eine Betriebstemperatur </= der Raumtemperatur von ca. 20°C zu wählen, insbesondere < 10 °C bzw. die Laserdiode auf ihre minimal erlaubte Betriebstemperatur abzukühlen.

Mit der erfindungsgemäßen Lösung wird ein SS-Interferometer-System zur Verfügung gestellt, welches auf einer VCSEL-Laserdiode basiert und für die Verwendung in der Ophthalmologie, insbesondere zur Bestimmung biometrischer Messwerte des Auges, geeignet ist.

Das vorliegende SS-OCT-System ist über einen breiten Wellenlängenbereich, bei einer hohen Kohärenzlänge und einer vergleichsweise hohen Wiederholrate, durchstimmbar und damit für biometrische Messungen des Auges insbesondere mittels Ganzaugenscans geeignet.

Die erfindungsgemäße Lösung zum Aufbau eines einfachen durchstimmbaren Diodenlasers beruht auf der Tatsache, dass bei einer Temperaturänderung im aktiven Lasermaterial eine Wellenlängenänderung erzielbar ist. Dabei kann diese Temperaturänderung indirekt durch den elektrischen Strompuls oder/und durch eine zusätzliche Heizung bzw. Abkühlung erfolgen.

Wie oben beschrieben, ist ein Temperaturgradient von ca. 0,1nm/K realisierbar und damit für einen Durchstimmbarkeit von 5nm eine Temperaturänderung von 50K erforderlich. Erfindungsgemäß ist vorgesehen diesen Gradienten auf >0,1nm/K, vorzugsweise auf ca. 0,3 bis 0,5nm/K einzustellen. Dies kann unter anderem durch die beschriebene Auswahl des aktiven Halbleiter-Lasermaterials, der Auswahl geeigneter Betriebsparameter und dem thermischen Management des Systems optimiert werden.

**Patentansprüche**

1. Verfahren zur Steuerung eines einfachen, für Ganzaugenscans geeignetes Halbleiterlaserdioden-basierten SS-Interferometer-Systems, bei dem mittels periodischer Strommodulation der Betrieb von Halbleiterlaserdioden so gestaltet wird, dass eine hochkohärente spektrale Laserlinie mit einer möglichst hohen Wiederholrate und über einen breiten Wellenlängenbereich durchstimmbar ist, **dadurch gekennzeichnet, dass** für biometrische Anwendungen am Auge folgende Parameter vorgesehen sind:

   - eine Mittenwellenlänge im Bereich von 600 - 1300nm,
   - eine Sweep Rate von 10kHz oder 100kHz,
   - eine Sweep Range im Bereich von 3 - 20nm und
   - eine optische Leistung am Auge im Bereich von 50 - 20000$\mu$W,
   - bei einer Kohärenzlänge von mindestens 20mm und
   - Strompulse mit einer Dauer von 1$\mu$s bei einer Wiederholrate von 100kHz oder einer Dauer von 10$\mu$s bei einer Wiederholrate von 10kHz realisiert werden.

2. Verfahren nach Anspruch 1, **dadurch gekennzeichnet, dass** die periodische Strommodulation unabhängig von der Richtung der Wellenlängenänderung erfolgt, so dass sowohl up- als auch down-Sweep Verwendung finden.

3. Verfahren nach Anspruch 1, **dadurch gekennzeichnet, dass** die Amplitude der Stromstöße in einem Bereich oberhalb des Schwellstromes $I_{th}$ liegen

4. Verfahren nach Anspruch 1, **dadurch gekennzeichnet, dass** die Dauer der Anstiegsflanke der Stromstöße < 500$\mu$s bis 50ns beträgt.

5. Verfahren nach Anspruch 1, **dadurch gekennzeichnet, dass** für die periodische Strommodulation Taktschwankungen von < 1ns, eine Wiederholbarkeit der Strompulse < 10ns und eine Amplitudenstabilität von < +/-5% zu gewährleisten sind.

6. Verfahren nach Anspruch 1, **dadurch gekennzeichnet, dass** ein Strompuls, basierend auf der Kenntnis der momentanen Wellenlänge mittels Feedback-System geregelt wird, wodurch eine bekannte Änderung der Wellenzahl über der Zeit realisiert wird.

7. Verfahren nach Anspruch 1, **dadurch gekennzeichnet, dass** eine lineare Änderung der Wellenzahl über der Zeit erfolgt.

8. Verfahren nach Anspruch 1, **dadurch gekennzeichnet, dass** das Feedback-System aus zwei unterschiedlichen fotosensitiven Materialien besteht.

9. Verfahren nach Anspruch 8, **dadurch gekennzeichnet, dass** ein nm/K- Gradient von > 0,1 nm/K, vorzugsweise zwischen 0,25 bis 0,5 nm/K eingestellt wird.

10. Verfahren nach einem der Ansprüche 1 bis 9, **dadurch gekennzeichnet, dass** als Halbleiterlaserdiode eine VCSEL-Laserdiode Verwendung findet.

**Claims**

1. Method for controlling a simple semiconductor-laser-diode-based SS-interferometer system suitable for whole-eye scans, in which, by means of periodic current modulation, the operation of semiconductor laser diodes is configured such that a highly coherent spectral laser line is tunable with as high a repetition rate as possible and over a wide wavelength range, **characterized in that** the following parameters are provided for biometric applications on the eye:

   - a centre wavelength in the range of 600 - 1300 nm,
   - a sweep rate of 10 kHz or 100 kHz,
   - a sweep range in the range of 3 - 20 nm and
   - an optical power at the eye in the range of 50-20 000 $\mu$W,
   - given a coherence length of at least 20 mm, and

- current pulses having a duration of 1 μs at a repetition rate of 100 kHz or a duration of 10 μs at a repetition rate of 10 kHz are realized.

2. Method according to Claim 1, **characterized in that** the periodic current modulation is effected independently of the direction of the wavelength change, such that both up-sweep and down-sweep are used.

3. Method according to Claim 1, **characterized in that** the amplitude of the current pulses is in a range above the threshold current $I_{th}$.

4. Method according to Claim 1, **characterized in that** the duration of the rising edge of the current pulses is < 500 μs to 50 ns.

5. Method according to Claim 1, **characterized in that** clock fluctuations of < 1 ns, a repeatability of the current pulses of < 10 ns and an amplitude stability of < +/- 5% are to be ensured for the periodic current modulation.

6. Method according to Claim 1, **characterized in that** a current pulse is controlled by means of a feedback system on the basis of the knowledge of the instantaneous wavelength, as a result of which a known change in the wavenumber over time is realized.

7. Method according to Claim 1, **characterized in that** a linear change in the wavenumber over time is effected.

8. Method according to Claim 1, **characterized in that** the feedback system consists of two different photosensitive materials.

9. Method according to Claim 8, **characterized in that** an nm/K gradient of > 0.1 nm/K, preferably between 0.25 and 0.5 nm/K, is set.

10. Method according to any of Claims 1 to 9, **characterized in that** a VCSEL laser diode is used as the semiconductor laser diode.

**Revendications**

1. Procédé de commande d'un système d'interféromètre SS à base de diodes laser à semiconducteur simple, adapté à des balayages de l'œil complet, dans lequel le fonctionnement de diodes laser à semiconducteur est configuré au moyen d'une modulation périodique du courant de sorte qu'une raie laser spectrale hautement cohérente puisse être accordée à une fréquence de répétition la plus élevée possible et sur une large gamme de longueurs d'onde, **caractérisé en ce que** les paramètres suivants sont prévus pour des applications de biométrie à l'œil :

   - une longueur d'onde centrale dans la plage de 600 - 1300 nm,
   - une vitesse de balayage de 10 kHz ou 100 kHz,
   - une étendue du balayage dans la plage de 3 - 20 nm, et
   - une puissance optique au niveau de l'œil dans la plage de 50 - 20000 μW,
   - avec une longueur de cohérence d'au moins 20 mm, et
   - des impulsions de courant d'une durée de 1 μs à une fréquence de répétition de 100 kHz ou d'une durée de 10 μs à une fréquence de répétition de 10 kHz sont réalisées.

2. Procédé selon la revendication 1, **caractérisé en ce que** la modulation périodique du courant s'effectue indépendamment de la direction de la modification de longueur d'onde, de sorte que des balayages aussi bien montants que descendants sont utilisés.

3. Procédé selon la revendication 1, **caractérisé en ce que** l'amplitude des impulsions de courant se situe dans une plage supérieure au courant de seuil $I_{th}$.

4. Procédé selon la revendication 1, **caractérisé en ce que** la durée du front montant des impulsions de courant est < 500 μs à 50 ns.

5. Procédé selon la revendication 1, **caractérisé en ce que** pour la modulation périodique du courant, des fluctuations

**EP 3 931 523 B1**

d'horloge de < 1 ns, une répétabilité des impulsions de courant < 10 ns et une stabilité d'amplitude < +/-5 % doivent être garanties.

6. Procédé selon la revendication 1, **caractérisé en ce qu'**une impulsion de courant est régulée au moyen d'un système de rétroaction sur la base de la connaissance de la longueur d'onde instantanée, réalisant ainsi une modification connue du nombre d'onde au cours du temps.

7. Procédé selon la revendication 1, **caractérisé en ce qu'**une modification linéaire du nombre d'onde au cours du temps est effectuée.

8. Procédé selon la revendication 1, **caractérisé en ce que** le système de rétroaction est constitué de deux matériaux photosensibles différents.

9. Procédé selon la revendication 8, **caractérisé en ce qu'**un gradient en nm/K de > 0,1 nm/K, de préférence compris entre 0,25 et 0,5 nm/K, est réglé.

10. Procédé selon l'une des revendications 1 à 9, **caractérisé en ce qu'**une diode laser VCSEL est utilisée en tant que diode laser à semiconducteur.

# Fig. 1

$$\frac{\Delta\lambda}{\Delta T} \sim 0{,}07 \ldots\ldots\ldots 0{,}25 \text{ mm/K}$$
(GaAs)    (AlGaInP)

Steuereinheit

VCSEL-Laserdiode

Temperaturmanagement
(aktive/passive Kühlung)

2

1

3

$\overline{T}$

optional

Anstiegszeit

4 optischer
Verstärker

optional

EP 3 931 523 B1

# Fig. 2

P/mW

5

Δλ (Durchstimmbereich)

λ/mm

L$_c$ (Kohärenzlänge)

spektrale Drift

P/mW    z.B.: 1 kHz , Tastverhältnis 30%

6

300µs

t/s

1ms

8      7

I

11

Retina

Kornea    Linse    Augenlänge

10
Nachbearbeitungseinheit
(Durchschnitt, Driftkompensation)

9

Biometer

EP 3 931 523 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008028312 A1
- WO 2018119077 A1
- US 8632181 B2, Bublitz

**In der Beschreibung aufgeführte Nicht-Patentliteratur**

- **COLE**. *Ultra-Widely Tunable VCSELs*, http://www. aomicro.com/tech/Cole_TUM_27_Sep_2012.pdf
- *DFB laser diodes from 760 nm to 830 nm*, https://nanoplus.com/fileadmin/user_upload/Data_sheets/nanoplus_DFB_760-830nm.pdf
- *760/763 nm single-mode VCSEL*, http://www.photonics.philips.com/pdf/ULM76X-SingleMode_TO5.pdf
- **MOON**. VCSEL-based swept source for low-cost optical coherence tomography. *Biomedical Optics Express*, 01 February 2017, vol. 8 (2), 1110-1121
- **HOGAN B.** Operation of VCSELs Under Pulsed Conditions. *VIXAR Application Note*, 21 January 2010
- **CHOI W et al.** Phase-sensitive swept-source optical coherence tomography imaging of the human retina with a vertical cavity surface-emitting laser light source. *Opt Lett*, 2013, vol. 38 (3), 338-340
- **GRULKOWSKI et al.** Retinal, anterior segment and full eye imaging using ultrahigh speed swept source OCT with vertical-cavity surface emitting lasers. *Biomedical Optics Express*, 01 November 2012, vol. 3 (11), 2733-2751